(19) Europäisches Patentamt
European Patent Office
Office européen des brevets

(11) EP 3 820 435 B1

(12) EUROPEAN PATENT SPECIFICATION

(45) Date of publication and mention of the grant of the patent:
**22.06.2022 Bulletin 2022/25**

(21) Application number: **19722924.8**

(22) Date of filing: **14.05.2019**

(51) International Patent Classification (IPC):
***A61K 8/46*** (2006.01) ***A61K 8/49*** (2006.01)
***A61Q 11/00*** (2006.01) ***A61K 8/81*** (2006.01)

(52) Cooperative Patent Classification (CPC):
**A61Q 11/00; A61K 8/463; A61K 8/494; A61K 8/8164; A61K 8/8176;** A61K 2800/434

(86) International application number:
**PCT/EP2019/062322**

(87) International publication number:
**WO 2020/011425 (16.01.2020 Gazette 2020/03)**

(54) **ORAL CARE COMPOSITION**

MUNDPFLEGEZUSAMMENSETZUNG

COMPOSITION D'HYGIÈNE BUCCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2018 PCT/CN2018/095646**
**09.08.2018 EP 18188166**

(43) Date of publication of application:
**19.05.2021 Bulletin 2021/20**

(73) Proprietors:
- **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO SE SI SK SM**
- **UNILEVER GLOBAL IP LIMITED**
**Wirral**
**Merseyside CH62 4ZD (GB)**
Designated Contracting States:
**CY DE GB IE IT MT RS TR**

(72) Inventors:
- **LI, Yajuan**
**Shanghai, Changning District 200335 (CN)**
- **YI, Shangchun**
**Shanghai, Changning District 200335 (CN)**

(74) Representative: **Tansley, Sally Elizabeth**
**Unilever PLC**
**Unilever Patent Group**
**Bronland 14**
**6708 WH Wageningen (NL)**

(56) References cited:
**EP-A1- 1 935 395** **WO-A1-2016/099524**

- **DATABASE GNPD [Online] MINTEL; 5 February 2018 (2018-02-05), anonymous: "CC Care Correction Bright Whitening Toothpaste", XP055552590, retrieved from www.gnpd.com Database accession no. 5429007**

EP 3 820 435 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to oral care compositions such as toothpastes, powders, gums, mouthwashes and the like. In particular, the present invention relates to oral care compositions comprising a pigment and a deposition aid for the pigment. The invention also relates to the use of such compositions for whitening the teeth of an individual.

### Background of the Invention

The enamel layer of the tooth is naturally an opaque white or slightly off-white colour. However, this enamel layer can become stained or discoloured. Many products we consume have a negative impact on our teeth and mouth. Many substances can stain or reduce the whiteness of one's teeth, in particular, certain foods, tobacco products, and fluids such as tea and coffee. These staining and discolouring substances are often able to permeate the enamel layer. This problem occurs gradually over many years, but imparts a noticeable discoloration of the enamel of one's teeth.

Consumers have always had a strong desire for whiter teeth and many individuals are dissatisfied with their current teeth colour. This desire for whiter teeth has given rise to a growing trend in the increased use of tooth whitening products which range from toothpastes to mouthwashes and chewing gums. The whitening effect can be produced by chemically altering or removing the stain and/or changing the visual perception of the color of the teeth.

It is known in the literature that the visual perception of a white substance can be altered through the deposition of an optical brightener, blue pigment or blue dye. Blue Covarine is a phthalocyanine blue pigment present in the toothpaste named white Now® and there have been attempts to enhance the deposition of this pigment on teeth for instant whitening benefits in oral care products.

Surfactants are commonly included in oral care products like toothpastes to help cleaning and cause foaming during use of the product, especially anionic surfactants. Foaming is experienced by the user that the product is effective in cleaning. However, the use of surfactants in oral care products prevents effective deposition of pigments on tooth surfaces due to their superior cleaning performance.

There is a need for an oral care composition which provides enhanced deposition of pigments in the presence of surfactants.

### Additional Information

WO 2016/099544 A1 (Colgate-Palmolive Company) discloses a tooth whitening oral care composition made by combining ingredients comprising flakes of a water soluble whitening film, a dye with a hue angle in the CIELAB system ranging from 200 to 320 degrees and an orally acceptable carrier vehicle.

EP 1 935 395 A1 (Unilever) discloses from 0.01 to 0.3% by weight of a pigment having a hue angle, h, in the CIELAB system of from 220 to 320 degrees, characterized in that the composition further comprises a soluble deposition aid for said pigment.

WO 2012/123241 A2 (Unilever) discloses an oral care composition suitable for delivering a temporary whitening effect to the surface of teeth, the composition comprising: a continuous phase comprising water or polyhydric alcohol or a mixture thereof; a particulate tooth surface whitening agent which is dispersed in the continuous phase, and a deposition aid for the particulate tooth surfaces whitening agent; characterised in that the deposition aid is a poly-(sulphonic acid) polymer. The particulate tooth whitening agent is a phthalocyanine blue pigment.

The additional information above does not describe an oral care composition comprising a copolymer of methyl vinyl ether and maleic anhydride, polyvinyl pyrrolidone, an ethoxylated alkyl sulfate anionic surfactant having a formula $RO(CH_2CH_2O)_nSO_3M$, wherein R is an alkyl or alkenyl group having from 8 to 18 carbon atoms, M is a solubilising cation comprising sodium, potassium, ammonium, substituted ammonium or mixtures thereof, n is the degree of ethoxylation of from 0.5 to 3, a pigment having a hue angle, h, in the CIELAB system of from 220 to 320 degrees, and a physiologically acceptable carrier, wherein the copolymer and the polyvinyl pyrrolidone are present in a weight ratio of from 1:20 to 1:4.

### Test and definitions

#### Dentifrice

"Dentifrice" for the purposes of the present invention means a paste, powder, liquid, gum or other preparation for cleaning the teeth or other surfaces in the oral cavity.

Toothpaste

"Toothpaste" for the purpose of the present invention means a paste or gel dentifrice for use with a toothbrush. Especially preferred are toothpastes suitable for cleaning teeth by brushing for about two minutes.

Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

Solubility

"Soluble" and "insoluble" for the purpose of the present invention, refers to the solubility of a source in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

Molecular Weight

"Molecular weight" for the purpose of the present invention, refers to the weight average molecular mass of a given polymer. The weight average molecular weight of polymers is determined by gel permeation chromatography against a polyethylene glycol standard.

Viscosity

Viscosity of a toothpaste is the value taken at room temperature (25°C) with a Brookfield Viscometer, Spindle No.93/94 and at a speed of 5 rpm for 1 minute. Values are quoted in centipoises (cP=mPa.s) unless otherwise specified.

Miscellaneous

Except in the examples, or where otherwise explicitly indicated, all numbers in this description indicating amounts of material or conditions of reaction, physical properties of materials and/or use may optionally be understood as modified by the word "about". All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

**Summary of the Invention**

In a first aspect, the present invention is directed to an oral care composition comprising:

a) a copolymer of methyl vinyl ether and maleic anhydride;

b) polyvinyl pyrrolidone;

c) an ethoxylated alkyl sulfate anionic surfactant having a formula $RO(CH_2CH_2O)_nSO_3M$, wherein R is an alkyl or alkenyl group having from 8 to 18 carbon atoms; M is a solubilising cation comprising sodium, potassium, ammonium, substituted ammonium or mixtures thereof; n is the degree of ethoxylation of from 0.5 to 3;

d) a pigment having a hue angle, h, in the CIELAB system of from 220 to 320 degrees; and

e) a physiologically acceptable carrier;

wherein the copolymer and the polyvinyl pyrrolidone are present in a weight ratio of from 1:20 to 1:4.

In a second aspect, the present invention is directed to a packaged oral care product comprising the oral care composition of the first aspect of this invention.

In a third aspect, the present invention is directed to a method of whitening teeth of an individual comprising the step of applying the oral care composition of any embodiment of the first aspect to at least one surface of the teeth of the individual.

All other aspects of the present invention will more readily become apparent upon considering the detailed description and examples which follow.

## Detailed Description of the Invention

The pigment according to the invention is a shade/material which is insoluble in the relevant medium, at the relevant temperature. This is in contrast to dyes which are soluble. The term "relevant medium", as used herein, refers to human saliva, the liquid medium in which the composition is used, at the temperature of the oral cavity during brushing of the teeth, i.e. up to 37°C. The relevant medium may also be water and the relevant temperature to be 25°C. The only limitation with respect to the pigment is that the same is suitable for use in the mouth.

The pigment has a hue angle, h, in the CIELAB system of from 220 to 320 degrees, more preferably from 250 to 290 degrees. A detailed description of hue angels may be found on p57 of Colour Chemistry 3rd edition by H. Zollinger published by Wiley-VCH. Preferably the pigment is violet or blue, more preferably the pigment is selected from one or more of those listed in the Colour Index International as pigment blue 1 through to pigment 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range. For example, such a hue angle may also be obtained by mixing a red and green-blue pigment to yield a blue or violet shaded pigment.

It is particularly preferred that the pigment is a blue pigment. Some examples of blue pigments suitable for use in this invention include inorganic blue pigments such as iron blue (C.I. 77510) and ultramarine blue (C.I. 77007). A preferred class of blue pigments suitable for use in the invention are organic blue pigments such as phthalocyanine blue pigments. Phthalocyanines are organometallic compounds containing four symmetrically arranged isoindole rings connected in a 16-membered ring linking with alternate carbon and nitrogen atoms. Most phthalocyanines contains a central, coordinated metal ion such as copper. Copper phthalocyanines have the basis structure:

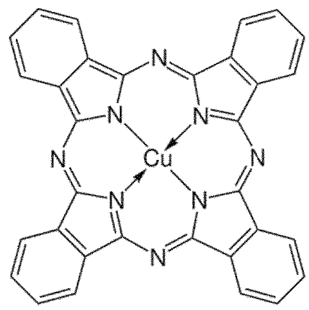

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule. The following pigments are illustrative phthalocyanine blue pigments preferably included in the composition according to the invention:

| C.I. Generic Name | C.I. Constitution Number | Crystal modification; Type | Number of halogen atoms |
|---|---|---|---|
| Pigment Blue 15 | 74160 | alpha | -- |
| Pigment Blue 15:1 | 74160 | alpha; phase stabilised | 0.5-1 Cl |
| Pigment Blue 15:2 | 74160 | alpha; phase & flocculation stabilised | 0.5-1 Cl |
| Pigment Blue 15:3 | 74160 | beta | -- |
| Pigment Blue 15:4 | 74160 | beta; flocculation stabilised | -- |
| Pigment Blue 15:6 | 74160 | epsilon | -- |
| Pigment Blue 16 | 74100 | gamma; metal-free | -- |

It is especially preferred that the pigment is phthalocyanine blue pigment selected from alpha copper phthalocyanines Pigment Blue 15, Pigment Blue 15:1, Pigment 15:2 and mixtures thereof. Most preferably the pigment is Pigment Blue 15:1. A commercially available example is Cosmenyl Blue A4R from Clariant.

The pigment suitable for use in this invention may also be mixtures of any of the above described materials.

The oral care composition of the present invention typically comprises from 0.001 to 1% by weight of the pigment, more preferably from 0.01 to 0.5%, and most preferably from 0.02 to 0.2%, based on total weight of the oral care composition and including all ranges subsumed therein.

The oral care composition of the invention comprises a deposition aid for the pigments. The term "deposition aid", as used herein, means a material which aids deposition of pigments from the continuous phase of the composition onto the surface of teeth during use of the composition. Use of the composition in the context of this invention typically involves application of the composition to the oral cavity, followed by brushing and/or rinsing. The deposition aid enhances deposition of the pigment onto the surfaces of teeth and thereby enhances the colour change caused by the pigment.

Suitable deposition aids for use in the invention include polymeric materials. The polymeric materials for use as deposition aids in the invention may be naturally or synthetically-derived. Preferably such polymeric materials are high molecular weight materials. "High molecular weight", as used herein, means the polymeric material has a molecular weight of at least 100,000 g/mol, preferably at least 200,000 g/mol.

Preferably, the deposition aid of the present invention includes copolymers of methyl vinyl ether and maleic anhydride, high molecular weight PEGs, high molecular weight cellulose ethers or mixtures thereof. Copolymers of methyl vinyl ether and maleic anhydride are particularly preferred, which are commercially available under the trade name GANTREZ®.

A particularly preferred example of such a polymer suitable for use in the present invention comprises:

$$-[-CH_2-CH(OCH_3)-CH(COOH)-CH(COOH)-]-$$

units in its structure, in which the-COOH groups are in free acid form. Such polymers may be linear or cross-linked. More preferably the polymer is linear. Polymers of this type are commercially available for example under the trade name GANTREZ® S (e.g. GANTREZ® S-96, GANTREZ® S-97).

The copolymer of methyl vinyl ether and maleic anhydride typically has a molecular weight of 500,000 g/mol to 2,000,000 g/mol, more preferably from 800,000 g/mol to 1,500,000 g/mol, and most preferably from 1,000,000 g/mol to 1,500,000 g/mol.

Alternative polymers which may be used comprise the units as described above in anhydride form, i.e. in which the two adjacent -COOH groups are cyclised to form a ring system. Such polymers are commercially available under the trade name GANTREZ® AN, e.g. GANTREZ® AN-119, GANTREZ® AN-903, GANTREZ® AN-139 and GANTREZ® AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the trade name GANTREZ® MS, e.g. GANTREZ® MS-955.

Other alternative polymers which may be used comprise the units as described above in partial ester form, for example in which some of the free -COOH groups are esterified with $C_{1-6}$ alkyl, e.g. ethyl or n-butyl. Such polymers are commercially available under the trade name GANTREZ® ES, e.g. GANTREZ® ES-225 or GANTREZ® ES-425.

Mixtures of any of the above described materials may also be used.

The copolymer of methyl vinyl ether and maleic anhydride is incorporated into the composition at preferably from 0.01 to 10%, more preferably at from 0.02 to 5% and most preferably at from 0.05 to 3% by weight, based on total

weight of the oral care composition.

The inventors have found unexpectedly that the incorporation of polyvinyl pyrrolidone into the composition can further enhance the deposition of pigments onto the surfaces of teeth. The polyvinyl pyrrolidone preferably has a molecular weight of from 100,000 g/mol to 2,000,000 g/mol, more preferably from 500,000 g/mol to 1,500,000 g/mol, and most preferably from 1,000,000 g/mol to 1,500, 000 g/mol.

The oral care composition of the present invention typically comprises from 0.1 to 15%, more preferably from 0.5 to 10% and most preferably from 1 to 10% by weight of the polyvinyl pyrrolidone, based on total weight of the oral care composition and including all ranges subsumed therein.

The weight ratio of the copolymer of methyl vinyl ether and maleic anhydride and the polyvinyl pyrrolidone are present in a weight ratio of from 1:20 to 1:4, preferably from 1:20 to 1:5, more preferably from 1:15 to 1:5 and most preferably from 1:10 to 1:5.

The oral care composition comprises an ethoxylated anionic surfactant which is an ethoxylated alkyl sulfate anionic surfactant having a formula $RO(CH_2CH_2O)_nSO_3M$, wherein R is an alkyl or alkenyl group having from 8 to 18 (preferably 12 to 18) carbon atoms, M is a solubilising cation comprising sodium, potassium, ammonium, substituted ammonium or mixtures thereof, n is the degree of ethoxylation of from 0.5 to 3, preferably from 1 to 3. Preferred ethoxylated alkyl sulfate anionic surfactant is sodium lauryl ether sulfate (SLES).

Typically, the ethoxylated alkyl sulfate anionic surfactant in oral care composition of the present invention ranges from 0.01 to 5%, more preferably from 0.05 to 3% and most preferably from 0.1 to 2%, based on total weight of the oral care composition and including all ranges subsumed therein.

The oral care composition may also comprise other surfactants. If other surfactants are present, it is preferred if the ethoxylated alkyl sulfate anionic surfactant is at least 75% by weight of the surfactant in the composition, more preferably from 80 to 100% and most preferably from 95 to 99% based on total weight of the surfactant in the composition. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include non-ionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Although other surfactants may be present, it is preferred if they are limited to levels below 0.5% by weight of the oral care composition, preferably less than 0.1% by weight of the oral care composition.

Preferably, the oral care composition has a pH from 4.0 to 10.0, more preferably from 5.0 to 9.0, and most preferably from 5.5 to 8.0.

The composition of the present invention is an oral care composition and typically comprises a physiologically acceptable carrier. The carrier preferably comprises at least thickener, humectant or a combination thereof.

Thickener may be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

In another especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

Thickener typically makes up from 0.01 to about 10%, more preferably from 0.1 to 9%, and most preferably, from 0.1 to 5% by weight of the oral care composition, based on total weight of the composition and including all ranges subsumed therein.

The oral care composition of the invention is preferably a toothpastes or gel. When the oral care composition is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 70% by weight of the composition, based on total weight of the composition and including all ranges subsumed therein.

The oral care composition of the present invention may contain a variety of other ingredients which are common in the art to enhance physical properties and performance, in addition to pigments which are included in the composition. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of whitening the teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for whitening the teeth of an individual. Alternatively and preferably, the use is non-therapeutic.

Typically, the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

## Examples

### Example 1

This example demonstrated the deposition of pigments by using a combination of polyvinyl pyrrolidone (PVP) and a copolymer of methyl vinyl ether and maleic anhydride. All ingredients are expressed by weight percent of the total formulation, and as level of active ingredient.

**TABLE 1**

| Ingredient | Samples | | | | |
|---|---|---|---|---|---|
| | 1 | 2 | 3 | 4 | 5 |
| Blue Covarine[a] (37.8%) | 0.075 | 0.075 | 0.075 | 0.075 | 0.075 |
| Gantrez S97 | 1.0 | -- | 1.0 | 1.0 | 1.0 |
| Polyvinyl pyrrolidone | -- | 5.0 | 0.5 | 5.0 | 10.0 |
| Water | Balance | Balance | Balance | Balance | Balance |
| a. Dispersion of C.I. 74160 (pigment blue 15:1) in water/glycerol under the trade name Cosmenyl Blue A4R from Clariant. | | | | | |

*Methods*

To evaluate the deposition of pigments, the following in vitro test was performed. The colour difference expressed as the CIELAB delta E (ΔE) value was calculated based on L*, a*, b* values measured using DigiEye (VeriVide, England) with the following formula:

$$\Delta E = \sqrt{\Delta L^2 + \Delta a^2 + \Delta b^2}$$

The test sample was diluted three times with water to form a slurry. Hydroxyapatite (HAP) discs were used as substrates. The baseline L*, a*, b* of the HAP discs was measured by DigiEye (VeriVide, England). Six HAP discs were used for each sample. The HAP discs were brushed with the slurry for one minute on a WIRA® brushing machine equipped with toothbrushes. The load of the tooth brushing was 175 g and the automatic brushing operated at a speed of 150 rpm for 1min. Thereafter the HAP discs were rinsed three times each time with 20 mL water by the brushing machine. After this step the L*, a*, b* values of each HAP disc were re-measured based on which the delta E from baseline were calculated and statistically analyzed.

*Results*

The results are summarized in Table 2 (error represents standard deviation for duplicate measurements). The higher the delta E value the more deposition of the pigment.

**TABLE 2**

| | Samples | | | | |
|---|---|---|---|---|---|
| | 1 | 2 | 3 | 4 | 5 |
| Delta E | 2.610±0.683 | 2.121±0.785 | 1.401±0.191 | 11.947±0.500 | 13.252±2.192 |

Samples 4 and 5 comprising a smaller weight ratio of copolymer to PVP showed significantly higher ΔE values ($p<0.05$) compared to other samples, indicating better deposition of the pigment.

Example 2

This example demonstrated the effect of anionic surfactants on the deposition of pigments. All ingredients are expressed by weight percent of the total formulation and as level of active ingredient.

**TABLE 3**

| Ingredient | Samples | | | | |
|---|---|---|---|---|---|
| | 6 | 7 | 8 | 9 | 10 |
| Blue Covarine[a] (37.8%) | 0.075 | 0.075 | 0.075 | 0.075 | 0.075 |
| Sodium lauryl sulfate (30%) | 1.8 | 1.8 | -- | -- | -- |
| Sodium lauryl ether sulfate (30%) | -- | -- | 1.8 | 1.8 | 1.8 |
| Gantrez S97 | 1.0 | 1.0 | 1.0 | 1.0 | 1.0 |
| Polyvinyl pyrrolidone | -- | 5.0 | -- | 3.0 | 5.0 |
| Water | Balance | Balance | Balance | Balance | Balance |

*Methods*

The test sample was diluted three times with water to form a slurry. Hydroxyapatite (HAP) discs were used as substrates. The baseline L*, a*, b* of the HAP discs was measured by DigiEye (VeriVide, England). Six HAP discs were used for each sample. The HAP discs were brushed with the slurry for one minute on a WIRA® brushing machine equipped with toothbrushes. The load of the tooth brushing was 175 g and the automatic brushing operated at a speed of 150 rpm for 1min. Thereafter the HAP discs were rinsed one time with 50 mL water by the brushing machine. After

this step the L*, a*, b* values of each HAP disc were re-measured based on which the delta E from baseline were calculated and statistically analyzed.

*Results*

The results are summarized in Table 4 (error represents standard deviation for duplicate measurements).

**TABLE 4**

| | Samples | | | | |
|---|---|---|---|---|---|
| | 6 | 7 | 8 | 9 | 10 |
| Delta E | 26.245±1.836 | 26.781±3.277 | 27.100±4.319 | 32.170±2.093 | 45.108±1.301 |

Samples 6 and 7 comprising sodium lauryl sulfate showed comparable ΔE values. But Sample 10 (consistent with the invention) comprising sodium lauryl ether sulfate showed significantly higher ΔE values ($p<0.01$) compared to other samples.

## Claims

1. An oral care composition comprising:

   a) a copolymer of methyl vinyl ether and maleic anhydride;
   b) polyvinyl pyrrolidone;
   c) an ethoxylated alkyl sulfate anionic surfactant having a formula $RO(CH_2CH_2O)_nSO_3M$, wherein R is an alkyl or alkenyl group having from 8 to 18 carbon atoms; M is a solubilising cation comprising sodium, potassium, ammonium, substituted ammonium or mixtures thereof; n is the degree of ethoxylation of from 0.5 to 3;
   d) a pigment having a hue angle, h, in the CIELAB system of from 220 to 320 degrees; and
   e) a physiologically acceptable carrier;

   wherein the copolymer and the polyvinyl pyrrolidone are present in a weight ratio of from 1:20 to 1:4.

2. The oral care composition according to claim 1, wherein the copolymer of methyl vinyl ether and maleic anhydride and the polyvinyl pyrrolidone are present in a weight ratio of from 1:20 to 1:5, preferably from 1:15 to 1:5.

3. The oral care composition according to claim 1 or claim 2, wherein the copolymer of methyl vinyl ether and maleic anhydride has a molecular weight of 500,000 g/mol to 2,000,000 g/mol, preferably from 800,000 g/mol to 1,500,000 g/mol.

4. The oral care composition according to any of the preceding claims, wherein the copolymer is the free acid form of the copolymer of methyl vinyl ether and maleic anhydride.

5. The oral care composition according to any of the preceding claims, wherein the composition comprises from 0.01 to 10% by weight of the copolymer of methyl vinyl ether and maleic anhydride, preferably from 0.02 to 5%.

6. The oral care composition according to any of the preceding claims, wherein the polyvinyl pyrrolidone has a molecular weight of from 100,000 to 2,000,000 g/mol, preferably from 500,000 to 1,500,000 g/mol.

7. The oral care composition according to any of the preceding claims, wherein the composition comprises from 0.1 to 15% by weight of polyvinyl pyrrolidone, preferably from 0.5 to 10%.

8. The oral care composition according to any of the preceding claims, wherein the pigment has a hue angel, h, in the CIELAB system of from 250 to 290.

9. The oral care composition according to claim 8, wherein the pigment is a blue pigment, preferably a phthalocyanine blue pigment.

**10.** The oral care composition according to claim 9, wherein the phthalocyanine blue pigment is selected from alpha copper phthalocyanines Pigment Blue 15, Pigment Blue 15:1, Pigment Blue 15:2 and mixtures thereof, preferably Pigment Blue 15:1.

**11.** The oral care composition according to any of the preceding claims, wherein the composition comprises from 0.001 to 1% by weight of the pigment, preferably from 0.01 to 0.5%.

**12.** The oral care composition according to any of the preceding claims, wherein the ethyoxylated alkyl sulfate anionic surfactant is sodium lauryl ether sulfate.

**13.** The oral care composition according to any of the preceding claims, wherein the composition comprises from 0.01 to 5% by weight of the ethyoxylated alkyl sulfate anionic surfactant, preferably from 0.05 to 3%.

**14.** The oral care composition according to any of the preceding claims, wherein the composition is a toothpaste or gel.

**15.** A method of whitening teeth of an individual comprising the steps of applying the composition according to any of the preceding claims to at least one surface of the teeth of an individual.

## Patentansprüche

**1.** Mundpflegezusammensetzung, umfassend:

a) ein Copolymer von Methylvinylether und Maleinsäureanhydrid;
b) Polyvinylpyrrolidon;
c) ein anionisches ethoxyliertes Alkylsulfat-Tensid mit einer Formel $RO(CH_2CH_2O)_nSO_3M$, worin R eine Alkyl- oder Alkenylgruppe mit 8 bis 18 Kohlenstoffatomen ist; M ein löslich machendes Kation ist, umfassend Natrium, Kalium, Ammonium, substituiertes Ammonium oder Mischungen davon; n der Ethoxylierungsgrad von 0,5 bis 3 ist;
d) ein Pigment mit einem Farbtonwinkel h im CIELAB-System von 220 bis 320 Grad; und
e) einen physiologisch verträglichen Träger;

wobei das Copolymer und das Polyvinylpyrrolidon in einem Gewichtsverhältnis von 1:20 bis 1:4 stehen.

**2.** Mundpflegezusammensetzung nach Anspruch 1, wobei das Copolymer von Methylvinylether und Maleinsäureanhydrid und das Polyvinylpyrrolidon in einem Gewichtsverhältnis von 1:20 bis 1:5, vorzugsweise von 1:15 bis 1:5, vorliegen.

**3.** Mundpflegezusammensetzung nach Anspruch 1 oder Anspruch 2, wobei das Copolymer von Methylvinylether und Maleinsäureanhydrid ein Molekulargewicht von 500.000 g/mol bis 2.000.000 g/mol, vorzugsweise von 800.00 g/mol bis 1.500.000 g/mol, aufweist.

**4.** Mundpflegezusammensetzung nach einem der vorhergehenden Ansprüche, wobei das Copolymer die freie Säureform des Copolymers von Methylvinylether und Maleinsäureanhydrid ist.

**5.** Mundpflegezusammensetzung nach einem der vorhergehenden Ansprüche, wobei die Zusammensetzung 0,01 bis 10 Gewichts-%, vorzugsweise 0,02 bis 5 Gewichts-%, des Copolymers von Methylvinylether und Maleinsäureanhydrid umfasst.

**6.** Mundpflegezusammensetzung nach einem der vorhergehenden Ansprüche, wobei das Polyvinylpyrrolidon ein Molekulargewicht von 100,.000 bis 2.000.000 g/mol, vorzugsweise von 500.000 bis 1.500.000 g/mol, aufweist.

**7.** Mundpflegezusammensetzung nach einem der vorhergehenden Ansprüche, wobei die Zusammensetzung 0,1 bis 15 Gewichts-%, vorzugsweise 0,5 bis 10 Gewichts-% Polyvinylpyrrolidon, umfasst.

**8.** Mundpflegezusammensetzung nach einem der vorhergehenden Ansprüche, wobei das Pigment einen Farbtonwinkel h in dem CIELAB-System von 250 bis 290 aufweist.

9. Mundpflegezusammensetzung nach Anspruch 8, wobei das Pigment ein blaues Pigment, vorzugsweise ein Phthalocyaninblau-Pigment, darstellt.

10. Mundpflegezusammensetzung nach Anspruch 9, wobei das Phthalocyaninblau-Pigment ausgewählt ist aus alpha-Kupferphthalocyanin-Pigment Blue 15, Pigment Blue 15:1, Pigment Blue 15:2 und Mischungen davon, vorzugsweise Pigment Blue 15:1.

11. Mundpflegezusammensetzung nach einem der vorhergehenden Ansprüche, wobei die Zusammensetzung 0,001 bis 1 Gewichts-%, vorzugsweise 0,01 bis 0,5 Gewichts-%, des Pigments umfasst.

12. Mundpflegezusammensetzung nach einem der vorhergehenden Ansprüche, wobei das anionische ethoxylierte Alkylsulfat-Tensid Natriumlaurylethersulfat ist.

13. Mundpflegezusammensetzung nach einem der vorhergehenden Ansprüche, wobei die Zusammensetzung 0,01 bis 5 Gewichts-%, vorzugsweise 0,05 bis 3 Gewichts-%, des anionischen ethoxylierten Alkylsulfat-Tensids umfasst.

14. Mundpflegezusammensetzung nach einem der vorhergehenden Ansprüche, wobei die Zusammensetzung eine Zahnpasta oder ein Gel ist.

15. Verfahren zum Aufhellen von Zähnen eines Individuums, umfassend die Schritte des Auftragens der Zusammensetzung nach einem der vorhergehenden Ansprüche auf mindestens eine Oberfläche der Zähne eines Individuums.

## Revendications

1. Composition pour le soin oral comprenant :

   a) un copolymère de méthylvinyléther et d'anhydride maléique ;
   b) de la polyvinylpyrrolidone ;
   c) un tensioactif anionique de sulfate d'alkyle éthoxylé ayant une formule $RO(CH_2CH_2O)_nSO_3M$, où R est un groupe alkyle ou alcényle ayant de 8 à 18 atomes de carbone ; M est un cation de solubilisation comprenant du sodium, potassium, ammonium, ammonium substitué ou mélanges de ceux-ci ; n est le degré d'éthoxylation de 0,5 à 3 ;
   d) un pigment ayant un angle de teinte, h, dans le système CIELAB de 220 à 320 degrés ; et
   e) un support physiologiquement acceptable ;

   où le copolymère et la polyvinylpyrrolidone sont présents dans un rapport de masse de 1:20 à 1:4.

2. Composition pour le soin oral selon la revendication 1, où le copolymère de méthylvinyléther et d'anhydride maléique et la polyvinylpyrrolidone sont présents dans un rapport de masse de 1:20 à 1:5, de préférence de 1:15 à 1:5.

3. Composition pour le soin oral selon la revendication 1 ou revendication 2, où le copolymère de méthylvinyléther et d'anhydride maléique présente une masse moléculaire de 500 000 g/mol à 2 000 000 g/mol, de préférence de 800 000 g/mol à 1 500 000 g/mol.

4. Composition pour le soin oral selon l'une quelconque des revendications précédentes, où le copolymère est la forme d'acide libre du copolymère de méthylvinyléther et d'anhydride maléique.

5. Composition pour le soin oral selon l'une quelconque des revendications précédentes, où la composition comprend de 0,01 à 10 % en masse du copolymère de méthylvinyléther et d'anhydride maléique, de préférence de 0,02 à 5 %.

6. Composition pour le soin oral selon l'une quelconque des revendications précédentes, où la polyvinylpyrrolidone présente une masse moléculaire de 100 000 à 2 000 000 g/mol, de préférence de 500 000 à 1 500 000 g/mol.

7. Composition pour le soin oral selon l'une quelconque des revendications précédentes, où la composition comprend de 0,1 à 15 % en masse de polyvinylpyrrolidone, de préférence de 0,5 à 10 %.

8. Composition pour le soin oral selon l'une quelconque des revendications précédentes, où le pigment présente un

angle de teinte, h, dans le système CIELAB de 250 à 290.

**9.** Composition pour le soin oral selon la revendication 8, où le pigment est un pigment bleu, de préférence un pigment bleu de phtalocyanine.

**10.** Composition pour le soin oral selon la revendication 9, où le pigment bleu de phtalocyanine est choisi parmi le Bleu Pigment 15, Bleu Pigment 15:1, Bleu Pigment 15:2 de phtalocyanines de cuivre alpha et mélanges de ceux-ci, de préférence le Bleu Pigment 15:1.

**11.** Composition pour le soin oral selon l'une quelconque des revendications précédentes, où la composition comprend de 0,001 à 1 % en masse du pigment, de préférence de 0,01 à 0,5 %.

**12.** Composition pour le soin oral selon l'une quelconque des revendications précédentes, où le tensioactif anionique de sulfate d'alkyle éthoxylé est le lauryléthersulfate de sodium.

**13.** Composition pour le soin oral selon l'une quelconque des revendications précédentes, où la composition comprend de 0,01 à 5 % en masse du tensioactif anionique de sulfate d'alkyle éthoxylé, de préférence de 0,05 à 3 %.

**14.** Composition pour le soin oral selon l'une quelconque des revendications précédentes, où la composition est un dentifrice ou gel.

**15.** Procédé de blanchiment des dents d'un individu comprenant les étapes d'application de la composition selon l'une quelconque des revendications précédentes sur au moins une surface des dents d'un individu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016099544 A1
- EP 1935395 A1
- WO 2012123241 A2


### Non-patent literature cited in the description


- **H. ZOLLINGER.** Colour Chemistry. Wiley-VCH
- *CHEMICAL ABSTRACTS,* 9004-32-4
- *CHEMICAL ABSTRACTS,* 9063-87-0